# EUROPEAN PATENT APPLICATION

(11) **EP 4 840 872 A1**
(43) Date of publication of application: **18.11.2026**
(21) Application number: 25869577.4
(22) Date of filing: 27.06.2025
(51) Int. Cl.: C07J 9/00, C07D 311/40, A61P 1/16

(54) **COMPOUND HAVING EFFECT OF DRASTIC PURGATION FOR ELIMINATING RETAINED FLUID, AND PREPARATION METHOD THEREFOR AND USE THEREOF**

(30) Priority: 25.06.2025 CN 202510856631
(71) Applicant: SICHUAN LANYING PHARMACEUTICAL CO., LTD., Leshan, Sichuan 614200 (CN)
(72) Inventor: The designation of the inventor has not yet been filed
(74) Representative: Patrade A/S
(86) International application number: PCT/CN2025/104442
(87) International publication number: WO 2026/148803

(57) **Abstract**

Provided is a drastic water-expelling compound having the chemical structure shown in formula (I). This compound is a novel pharmaceutical natural product of tetracyclic triterpenoids. It exhibits favorable lipophilicity and chemical stability, can be conveniently extracted from the rhizomes of the herbal plant *Begonia limprichtii Irmscher,* and can be formulated into oral or injectable dosage forms. Further provided is a method for preparing said compound and use thereof. The compounds of the present invention significantly inhibit the increase in mouse abdominal volume and ascites weight in a dose-dependent manner, demonstrating their potential as a highly promising novel drugs for draining ascites.

## Description

### TECHNICAL FIELD

The present invention relates to the field of medicine, and specifically to a novel pharmaceutical natural product of tetracyclic triterpenoids. More specifically, it relates to a structure of Daoshuilianin A, extraction method and use thereof.

### BACKGROUND

Ascites refers to the abnormal accumulation of fluid in the abdominal cavity caused by various factors. Its pathogenesis is a complex process involving multiple organs and systems. The main factors include: liver cirrhosis leading to portal hypertension and hypoalbuminemia, which is the most common cause of ascites; congestive heart failure, pericarditis, etc., which impede venous return; nephrotic syndrome resulting in retention of water and waste in the body; tumors such as ovarian cancer and colon cancer, compressing lymphatic vessels and obstructing lymphatic return; tuberculous peritonitis and inflammatory bowel disease, etc., increasing the permeability of peritoneal blood vessels; malnutrition, especially hypoalbuminemia, causing fluid leakage into the abdominal cavity; and other factors such as trauma, acute pancreatitis, connective tissue diseases, etc.

The harms of ascites to patients are manifested in the following reasons: (1) Direct impact on the body: Ascites may cause abdominal distension and pain, and in severe cases, even lead to dyspnea, impairing the patient's mobility and daily life. (2) Increased burden on the circulatory system: After ascites formation, the leakage of a large amount of fluid into the abdominal cavity leads to hemoconcentration, reduced effective circulating blood volume, increased blood viscosity, and slowed blood flow, which can cause hypotension or shock. (3) Kidney damage: Ascites contains a large amount of nutrients and albumin. Massive protein loss can exacerbate the patient's hypoalbuminemia, further leading to changes in the distribution of circulatory blood volume and damaging the kidneys. (4) Increased risk of infection: The presence of ascites makes it easy for bacterial infection within the abdominal cavity, especially during abdominal paracentesis or drainage of ascites, which is more likely to introduce secondary infection and lead to peritonitis. (5) Increased additional burden on the liver: The formation of ascites exacerbates liver ischemia and hypoxia, aggravating hepatocellular necrosis. Especially for patients with cirrhosis, the onset of ascites usually indicates that the liver has progressed to the decompensated stage. (6) Other complications: These such as hepatic encephalopathy, esophageal and gastric variceal bleeding, etc., can endanger the patient's life.

Timely symptomatic treatment of ascites to promote the fluid drainage from the abdominal cavity can not only alleviate the patient's suffering but also prevent complications, which is beneficial to the patient's health. Current treatment approaches for ascites, generally include salt restriction, sodium excretion, and diuresis. For refractory ascites and tense ascites, methods such as paracentesis, albumin infusion, transjugular intrahepatic portosystemic shunt, and liver transplantation can be used. However, the efficacy of these methods is not ideal. Traditional Chinese medicine (TCM) has a therapeutic method called "drastic water-expelling method," utilizing classic herbs including Gansui (*Euphorbia kansui*), Daji (*Euphorbia pekinensis*), and Badou (*Croton tiglium*), which can promote rapid fluid expulsion through the intestinal tract, showing significant clinical efficacy with distinct TCM characteristics. The problems are that these several Euphorbiaceae Chinese herbs are highly toxic, and their active components and mechanism for drastically water-expelling remain unclear, warranting further in-depth exploration.

Daoshuilian (*Begonia limprichtii Irmscher*) is a folk medicinal herbal in Southwest China. As a perennial herbaceous species of the family Begoniaceae, it has been employed to treat injuries from falls and blows, rheumatic pain, sore throat, etc., with a good safety. There have been no reports on the chemical constituents and water-expelling effect of this medicinal material.

### SUMMARY

To address the above-mentioned technical problems, it is an object of the present invention is to provide a small-molecule compound with a novel structure and a drastic water-expelling effect, serving as a novel therapeutic alternative drug for draining ascites.

To achieve the above object, the present invention provides the following technical solutions:
In a first aspect, the present invention provides a drastic water-expelling compound having a chemical structural formula as shown in Formula (I):

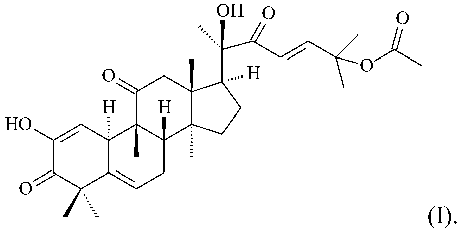

The aforementioned compound, named "Daoshuilianin A", features a parent nucleus containing 30 carbon atoms, making it a typical tetracyclic triterpenoid. Its structural feature is characterized by the attachment of the angular methyl group 19-CH₃ to C-9, rather than the conventional C-10 position in triterpenoids. While it also belongs to the cucurbitacins, it lacks the hydroxyl substitution at C-16 commonly found in cucurbitacins, thus representing a rare 16-dehydroxycucurbitacin. This novel structure has not been reported previously. Daoshuilianin A is a white powder with a bitter taste, sparingly soluble in water, freely soluble in organic solvents such as methanol, ethanol, ethyl acetate, and chloroform. Stability studies demonstrated that no reduction in its content and no alterations in properties are observed after 12 months of storage at room temperature. Under accelerated testing conditions of 40°C and 75% relative humidity for 6 months, no significant alterations in various physicochemical properties were observed either. These indicate that the compound possesses high chemical stability, providing favorable conditions for the manufacture, transport, and storage of its pharmaceutical preparations.

In a second aspect, it is another object of the present invention to provide a pharmaceutical composition comprising the compound of the present invention.

Preferably, said pharmaceutical composition further comprises a pharmaceutically acceptable carrier and/or excipient.

In a third aspect, it is another object of the present invention to provide a method for preparing the compound of the present invention, comprising the following steps:
a. Providing dried rhizomes of *Begonia limprichtii Irmscher*, pulverizing them, extracting with an organic solvent, filtering, and concentrating to obtain a fluid extract;
b. Adding the fluid extract to water, stirring, filtering, washing with water, and drying to obtain a precipitate;
c. Separating the precipitate by silica gel column chromatography, eluting with a gradient of petroleum ether-ethyl acetate;
d. Monitoring the fractions by silica gel column chromatography, combining, and concentrating to obtain the compound.

Preferably, the organic solvent in step (a) may be any one selected from methanol, ethanol, ethyl acetate, acetone, chloroform, petroleum ether, or a mixture thereof. Exemplary mixture include such as a combination of petroleum ether and ethyl acetate (e.g., in a volume ratio of 2:1). More preferably, the organic solvent is lower alcohol, such as methanol or ethanol. and even more preferably, pharmaceutical alcohol, i.e., 75% to 85% (v/v) aqueous ethanol, to comply with relevant pharmaceutical production regulations, cost-effectiveness, safety, and environmental friendliness. The extraction method may be performed by percolation, reflux, maceration, microwave, or supercritical extraction, preferably by reflux.

Preferably, the weight-to-volume ratio of *Begonia limprichtii Irmscher* to the organic solvent is 1 g : 3 to 5 mL, more preferably 1 g : 4 mL.

Preferably, step (b) is a water precipitation process, wherein water is added in an amount of 5 to 15 times the weight of the extract, preferably 10 times. The mixture is stirred at room temperature or under heating to fully dissolve and suspend the paste. Subsequently, the mixture is cooled, allowed to stand, and then filtered. The precipitate is washed repeatedly with small portions of water.

Preferably, the column chromatography in step (c) may be repeated until the target compound with the desired purity is obtained.

Preferably, in said step (d), the column chromatography fractions are monitored by thin-layer chromatography (TLC) or high-performance liquid chromatography (HPLC).

More preferably, said thin-layer chromatography is performed on conventional silica gel plates, using petroleum ether-ethyl acetate (3:1) or chloroform-methanol (98:2) as the developing solvent system, with visualization by iodine vapor staining method.

More preferably, the liquid chromatography method uses a conventional C18 reversed-phase silica gel column, with a mobile phase of methanol-0.1% formic acid (50:50 to 100:0) under gradient elution, and a detection wavelength of 237 nm.

The preparation method of the present invention features a short extraction process, simple operation, and low cost, with column chromatography packing being reusable, thereby making it feasible for scaled-up production.

In a fourth aspect, it is an object of the present invention to provide use of the compound or pharmaceutical composition of the present invention in the preparation of a medicament for draining ascites.

It can be seen that when evaluating the ascites-draining efficacy of Daoshuilianin A, the results show that, in mice inoculated with H22 hepatoma cells, the abdominal volume increased significantly; however, oral administration of Daoshuilianin A (at doses of 10-40 mg·kg⁻¹·d⁻¹) by gauge significantly inhibits the increase in both abdominal volume and ascites weight in a dose-dependent manner. Concurrently, it can be observed that mice in the treatment groups exhibit increased frequency of defecation and loose stools, manifesting obvious diarrhea. This effect is analogous to the Traditional Chinese Medicine (TCM) therapeutic principle of "drastic water-expelling method."

Preferably, the compound is formulated into a pharmaceutically acceptable dosage form either as a pure compound or as a plant extract containing such compound.

Preferably, the dosage form comprises a pharmaceutically acceptable carrier and/or excipient.

Pure Daoshuilianin A or plant extracts containing Daoshuilianin A can be prepared into various pharmaceutically acceptable dosage forms to meet the diverse requirements of clinical medication. These dosage forms include, but are not limited to, tablets, hard capsules, soft capsules, granules, dropping pills, ointments, and injections. The pharmaceutically acceptable carriers, excipients, or additives required for preparations specifically include, for example, pharmaceutically commonly used diluents and absorbents such as starch, dextrin, calcium sulfate, lactose, mannitol, sucrose, sodium chloride, glucose, calcium carbonate, kaolin, microcrystalline cellulose, aluminum silicate, calcium hydrogen phosphate, light magnesium oxide, etc.; pharmaceutically commonly used wetting agents and binders such as water, glycerin, polyethylene glycol, ethanol, propanol, starch paste, dextrin, syrup, honey, glucose solution, gum arabic mucilage, gelatin mucilage, sodium carboxymethyl cellulose, shellac, methyl cellulose, potassium phosphate, polyvinylpyrrolidone, etc.; pharmaceutically commonly used disintegrants such as dry starch, alginates, agar powder, laminarin starch, sodium bicarbonate, citric acid, calcium carbonate, polyoxyethylene, sorbitan fatty acid esters, sodium dodecyl sulfonate, methyl cellulose, ethyl cellulose, etc.; disintegration inhibitors such as sucrose, glyceryl tristearate, cocoa butter, hydrogenated oils, etc.; lubricants such as talc, silicon dioxide, corn starch, stearates, boric acid, liquid paraffin, polyethylene glycol, etc.; pharmaceutically commonly used co-solvents such as ethanol, propylene glycol, polyethylene glycol, polysorbate 80, lecithin, urea, sodium benzoate, sodium salicylate, nicotinamide, cyclodextrin, soybean oil, etc. Pharmaceutically acceptable carriers or excipients are not exhaustively listed herein, and one skilled in the art can select specifical ones based on common general knowledge.

Compared with the draining-ascites drug in the prior art, the present invention has the following beneficial effects:
(1) Daoshuilianin A is a novel small-molecule component with a structural type entirely distinct from existing water-expelling drugs, suggesting it may involve different targets and mechanisms of action;
(2) The ascites-draining effect of Daoshuilianin A resembles the Traditional Chinese Medicine (TCM) therapeutic principle of "drastic water-expelling method", promoting the rapid elimination of accumulated fluid via the intestines, thereby demonstrating significant efficacy;
(3) The source plant has a long history of use as a folk medical herb with no obvious toxic side effects, indicating that Daoshuilianin A possesses good clinical safety;
(4) The source plant is a herbaceous species of the genus *Begonia* Linn., similar to common horticultural begonia flower varieties. It can be artificially propagated and cultivated on a large scale, thereby meeting the demand for raw materials in mass production;
(5) The preparation process of Daoshuilianin A is simple, enabling to obtain high-purity compounds at low cost;
(6) Daoshuilianin A has strong lipophilicity and good solubility, facilitating easy oral absorption;
(7) Daoshuilianin A exhibits stable physicochemical properties and has a long shelf life.

In summary, the Daoshuilianin A of the present invention has a novel structure and outstanding advantages such as high efficiency and a novel mechanism. With readily available raw materials and low cost, it represents a novel draining-ascites drug with great development potential.

### BRIEF DESCRIPTION OF THE DRAWINGS

Figure 1 depicts the mass spectrum of Daoshuilianin A.
Figure 2 depicts the ¹H-NMR spectrum of Daoshuilianin A.
Figure 3 depicts the ¹³C-NMR spectrum of Daoshuilianin A.
Figure 4 depicts the HPLC chromatogram of Daoshuilianin A.
Figure 5 depicts the ultraviolet spectrum of Daoshuilianin A.
Figure 6 depicts the efficacy of Daoshuilianin A in draining mouse ascites, wherein: (A) a comparison of the general appearance of the mice; (B) a comparison of body weight; and (C) a comparison of abdominal circumference.

### DETAILED DESCRIPTION OF THE INVENTION

The technical solutions of the present invention are further described below with reference to the accompanying drawings and specific embodiments. Unless otherwise specified, the instruments or reagents used in the examples are conventional instruments or reagents in the art, which are commercially available conventional products. Unless otherwise specified, the specific experimental operations involved herein are understandable or known to those of one skilled in the art based on common general knowledge or conventional technical means, and thus will not be redundantly described herein.

### Embodiment 1: Extraction, Isolation, and Structure Characterization of Daoshuilianin A 1. Example 1: Extraction and Isolation of Daoshuilianin A

(1) 300 g of dried rhizomes of *Begonia limprichtii Irmsch*. (known in Chinese as "Daoshuilian" or "Jiyeqiuhaitang"), purchased from a herbal market in Leshan City, Sichuan Province were crushed into coarse powder. The powder was placed in a round-bottom flask, extracted with 1.2 L of methanol under reflux for 1 hour, cooled, and filtered to obtain an extracting solution. The residue was subjected to the same extraction procedure two additional times and another two extracting solutions were obtained. The three extracting solutions were combined and concentrated under reduced pressure to yield 65 g of extract.
(2) The extract was poured into 650 mL of water, heated in a water bath at 80°C, and stirred rapidly to ensure full dissolution, dispersion, and suspension of the extract. The mixture was then cooled, allowed to stand for 1 hour, and filtered. The filter cake was washed sequentially with small portions of water and dried to obtain 33 g of precipitated powder.
(3) 20 g of the precipitated powder was dissolved in 60 mL of methanol, mixed with silica gel, and dried. The mixture was loaded onto a silica gel column (silica gel specification: 200-300 mesh) and eluted with a gradient of petroleum ether-ethyl acetate (10:0 to 0:10). A total of 80 fractions (Fr. 1 to Fr. 80) were collected. Each fraction was analyzed by silica gel thin-layer chromatography (TLC) using petroleum ether-ethyl acetate (3:1, v/v) as the developing system and visualized by iodine vapor staining. The results showed that Fractions 15 to 18 (Fr. 15 to Fr. 18) contained the same single major spot. These fractions were combined and concentrated to yield 1.8 g of white powder (designated as PSY-2).

The purity of PSY-2 was determined by high performance liquid chromatography (HPLC) under the following conditions: Column: ODS column (150 × 4.6 mm, 5 µm); Mobile Phase: Methanol and 0.1% formic acid, gradient elution from 50:50 to 100:0; Detection Wavelength: 237 nm; Flow Rate: 1.0 mL/min.

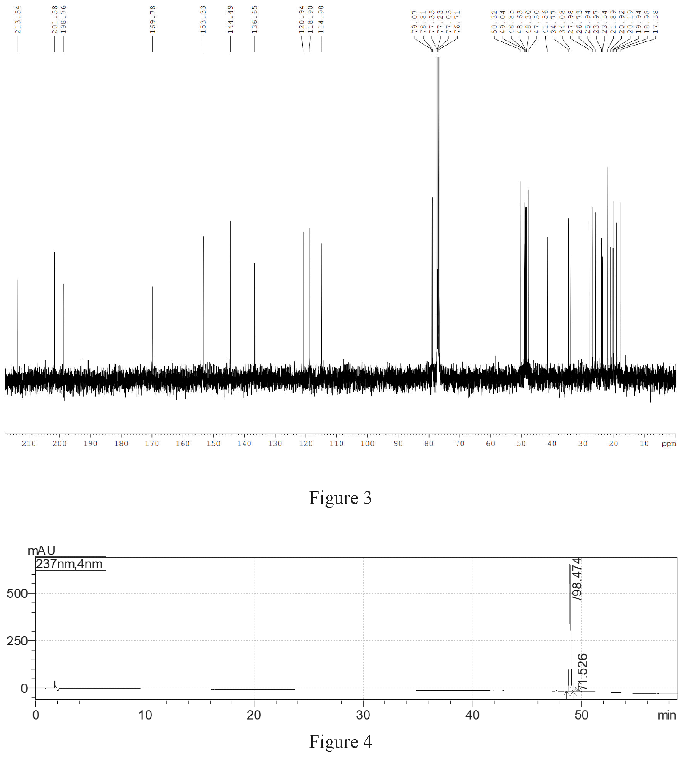

As shown in Figure 4, the chromatogram displayed a single major peak with a purity of >98%.

### 2. Structure Characterization

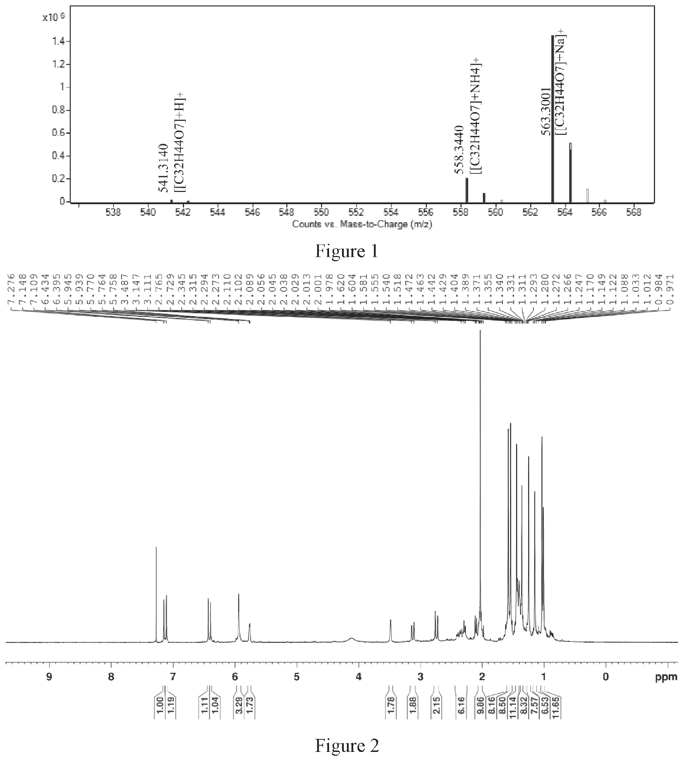

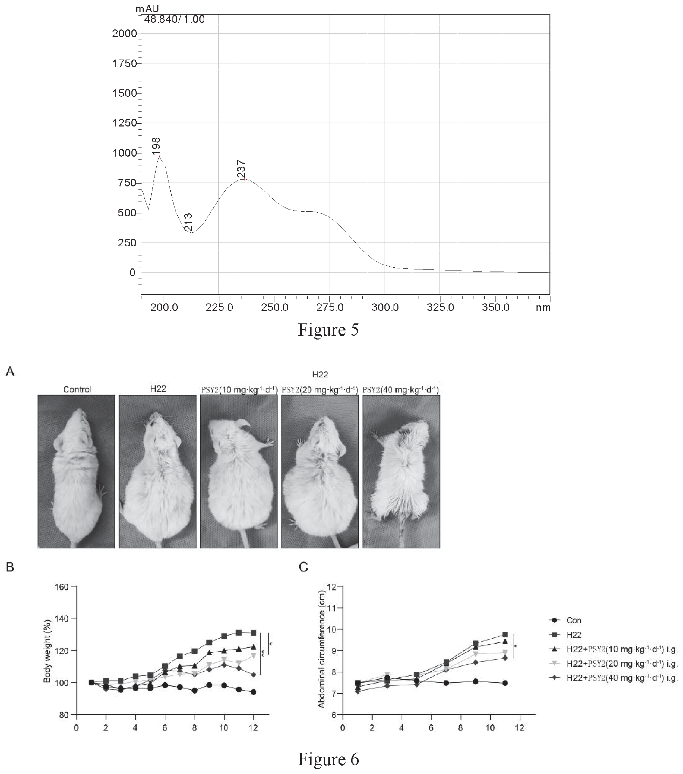

Pure Daoshuilianin A appeared as a white powder. [α]20,D +11.82° (c0.11, MeOH), UV λmax :237, 266 (sh) nm (Figure 5); ESI-TOF-MS m/z: 541.3140 [M+H]⁺, 558.3440 [M+NH₄]⁺, 563.3001 [M+Na]⁺ (Calculated for C₃₂H₄₄O₇, 540.308704) (Figure 1).

¹H-NMR(400MHz, CDCl₃) δppm: 1.03 (3H, s, H-18), 1.09 (3H, s, H-19), 1.15 (3H, s, H-29), 1.25 (3H, s, H-28), 1.36 (3H, s, H-30), 1.44 (3H, s, H-21), 1.54 (3H, s, H-26), 1.58 (3H, s, H-27), 2.03 (3H, s, Ac), 5.76 (1H, m, H-6), 5.94 (1H, d, J=2.4Hz, H-1), 6.41 (1H, d, J=15.6Hz, H-23), 7.12 (1H, d, J=15.6Hz, H-24), 3.49 (1H, br s, H-10), 3.13 (1H, d, J=14.4Hz, H12b), 2.74 (1H, d, J=14.4Hz, H12a), 2.33 (1H, m, H-7a), 2.03 (1H, m, H-7b), 2.04 (1H, m, H-8), 0.9~1.7 (5H, m, H-15,16,17) (Figure 2).

¹³C-NMR(100MHz, CDCl₃) δppm: 114.98 (C-1), 144.49 (C-2), 198.76 (C-3), 47.50 (C-4), 136.65 (C-5), 120.94 (C-6), 23.54 (C-7), 41.56 (C-8), 48.85 (C-9), 34.77(C-10), 213.54(C-11), 48.63(C-12), 50.32(C-13), 48.30(C-14), 34.08(C-15), 20.92(C-16), 49.04(C-17), 18.98(C-18), 19.94(C-19), 78.81(C-20), 23.97(C-21), 201.58(C-22), 118.90(C-23), 153.33(C-24), 79.07(C-25), 26.73(C-26), 25.94(C-27), 27.98(C-28), 20.19(C-29), 17.58(C-30), 21.89(Ac), 169.78(Ac) (Figure 3).

Based on the analysis of 2D spectra including HSQC and HMBC and with reference to relevant literature (Magn. Reson. Chem. 2005, 43, 489-491), the above spectral data were fully assigned. PSY-2 was identified as a novel cucurbitacin and named Daoshuilianin A.

The molecular formula of Daoshuilianin A (designated as PSY-2) is C₃₂H₄₄O₇, with a molecular weight of 540. Its molecular structure is shown in Formula (I) below.

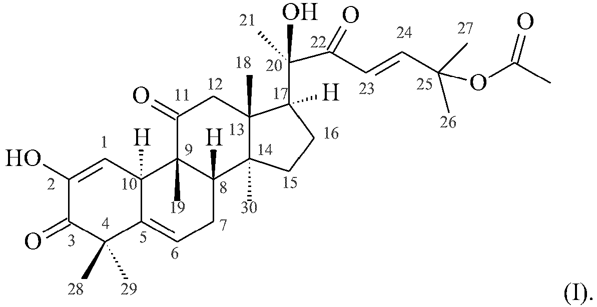

The systematic name of Daoshuilianin A is (6R,E)-6-hydroxy-6-((9R,10R,13R,14S,17S)-2-hydroxy-4,4,9,13,14-pentamethyl-3,11-dioxo-4,7,8,9,10,11,12,13,14,15,16,17-dodecahydro-3H-cyclopenta[a]phenanthren-17-yl)-2-methyl-5-oxohept-3-en-2-yl acetate.

### 3. Example 2: Extraction and Isolation of Daoshuilianin A

(1) 100 g of dried *Begonia limprichtii Irmscher* (Daoshuilian) was crushed into coarse powder and placed in a round-bottom flask. 500 mL of 80% (v/v) ethanol was added, and the mixture was extracted under reflux for 1 hour. After cooling, the mixture was filtered to obtain an extracting solution. The residue was subjected to the same extraction procedure two additional times and another two extracting solutions were obtained. The three extracting solutions were combined and concentrated under reduced pressure to yield 22 g of extract.
(2) The extract was poured into 330 mL of water, heated in a water bath at 80°C, and stirred rapidly to ensure full dissolution, dispersion, and suspension of the extract. The mixture was then cooled, allowed to stand for 1 hour, and filtered. The filter cake was washed sequentially with small portions of water and dried to obtain 13 g of precipitated powder.
(3) 10 g of the precipitated powder was dissolved in 30 mL of methanol, mixed with silica gel, and dried. The mixture was loaded onto a silica gel column (silica gel specification: 200-300 mesh) and eluted with a gradient of petroleum ether-ethyl acetate (10:0 to 0:10). Each fraction was analyzed by silica gel thin-layer chromatography (TLC) using petroleum ether-ethyl acetate (3:1, v/v) as the developing system and visualized by iodine vapor staining. Based on the TLC results, appropriate fractions were combined and concentrated to yield 1.2 g of pale yellow powder.
(4) The obtained powder was dissolved in 5 mL of methanol, mixed with silica gel, and dried. The mixture was loaded onto a silica gel column (silica gel specification: 200-300 mesh) and eluted with a gradient of petroleum ether-ethyl acetate (10:0 to 0:10). Guided by TLC analysis, the fractions were combined and concentrated to yield 0.7 g of white powder.

### 4. Example 3: Extraction and Isolation of Daoshuilianin A

(1) 100 g of dried *Begonia limprichtii Irmscher* was crushed into coarse powder and placed in a round-bottom flask. 300 mL of ethyl acetate was added, and the mixture was extracted under reflux for 1 hour. After cooling, the mixture was filtered to obtain an extracting solution. The residue was subjected to the same extraction procedure two additional times and another two extracting solutions were obtained. The three extracting solutions were combined and concentrated under reduced pressure to yield 13 g of extract.
(2) The extract was poured into 65 mL of water, heated in a water bath at 80°C, and stirred rapidly to ensure full dissolution, dispersion, and suspension of the extract. The mixture was then cooled, allowed to stand for 1 hour, and filtered. The filter cake was washed sequentially with small portions of water and dried to obtain 11 g of precipitated powder.
(3) 10 g of the precipitated powder was dissolved in 30 mL of methanol, mixed with silica gel, and dried. The mixture was loaded onto a silica gel column (silica gel specification: 200-300 mesh) and eluted with a gradient of petroleum ether-ethyl acetate (10:0 to 0:10). Each fraction was analyzed by silica gel TLC using petroleum ether-ethyl acetate (3:1, v/v) as the developing system and visualized by iodine vapor staining. The fractions were combined and concentrated to yield 0.9 g of white powder.

### 5. Example 4: Extraction and Isolation of Daoshuilianin A

(1) 100 g of dried *Begonia limprichtii Irmscher* was crushed into coarse powder and placed in a round-bottom flask. 350 mL of petroleum ether-ethyl acetate (2:1, v/v) was added, and the mixture was extracted under reflux for 1 hour. After cooling, the mixture was filtered to obtain an extracting solution. The residue was subjected to the same extraction procedure two additional times and another two extracting solutions were obtained. The three extracting solutions were combined and concentrated under reduced pressure to yield 12 g of extract.
(2) The extract was poured into 90 mL of water, heated in a water bath at 80°C, and stirred rapidly to fully dissolve, disperse, and suspend the extract. The mixture was then cooled, allowed to stand for 1 hour, and filtered. The filter cake was washed sequentially with small portions of water and dried to obtain 11 g of precipitated powder.
(3) 10 g of the precipitated powder was dissolved in 30 mL of methanol, mixed with silica gel, and dried. The mixture was loaded onto a silica gel column (silica gel specification: 200-300 mesh) and eluted with a gradient of petroleum ether-ethyl acetate (10:0 to 0:10). Each fraction was analyzed by silica gel TLC using petroleum ether-ethyl acetate (3:1, v/v) as the developing system and visualized by iodine vapor staining. The fractions were combined and concentrated to yield 1.0 g of white powder.

Chemical structure characterization confirmed that the products obtained from the extraction and isolation describes in Examples 2 to 4 above were identical to Daoshuilianin A (PSY-2).

### Embodiment 2: Pharmacodynamic Efficacy of Daoshuilianin A (PSY-2)

### 1. Experimental Methods:

BALB/c mice were intraperitoneally injected with H22 murine hepatoma cells (2 × 10⁶ cells/mouse) diluted in 200 µL of PBS. Seven days later, ascites was collected, and the ascites cells were resuspended in PBS to a concentration of 2.67 × 10⁷ /mL. Subsequently, each mouse received an intraperitoneal injection of H22 cells (2 × 10⁶ cells/mouse) diluted in 200 µL of PBS. Mice inoculated with the H22 cells were administered the test compound by oral gavage once daily, and changes in ascites volume were observed.

The BALB/c mice were randomly divided into five groups:
(1) Normal Control Group;
(2) Model Group: H22 inoculation only;
(3) Low-Dose PSY-2 Group: H22 + PSY-2 (10 mg·kg⁻¹·d⁻¹);
(4) Medium-Dose PSY-2 Group: H22 + PSY-2 (20 mg·kg⁻¹·d⁻¹);
(5) High-Dose PSY-2 Group: H22 + PSY-2 (40 mg·kg⁻¹·d⁻¹).

### 2. Observation Indicators:

Over a period of 12 days, mouse body weight was measured and recorded, and changes in abdominal circumference were monitored.

### 3. Experimental Results:

Mice inoculated with H22 cells exhibited significant abdominal enlargement. In contrast, oral administration of PSY-2 significantly inhibited the abdominal enlargement in a dose-dependent manner (Figure 6A).

Concurrently, measurements of body weight and abdominal circumference showed consistent trends. The body weight and abdominal circumference increased significantly in mice inoculated with H22 cells over the 12-day period. However, these increases were significantly suppressed in mice treated with PSY-2 (Figure 6B and 6C).

Furthermore, it was observed that mice in the drug-administration groups exhibited obvious diarrhea, characterized by increased defecation frequency and loose stools. This suggests that the ascites was primarily eliminated via the intestines, demonstrating characteristics consistent with the TCM therapeutic principle of "drastic water-expelling".

### Embodiment 3: Preparation of Daoshuilianin A Tablets

50 g of Daoshuilianin A was mixed with 1900 g of a filler mixture (weight ratio of lactose : starch = 3:2) in a high-shear mixer granulator. The mixture was first blended at low speed (approx. 100 to 200 rpm) for 5 to 10 minutes to ensure uniform dispersion of the active ingredient within the fillers. Then, 50 mL of a 5% (w/v) hydroxypropyl methylcellulose (HPMC) aqueous solution (binder) was added, and mixing continued at medium speed (approx. 300 to 400 rpm) for 10 to 15 minutes to form a soft mass. The mass was granulated through an 80-mesh sieve and dried to control moisture content between 2% and 5%. The dried granules were sized through a 100-mesh sieve. Finally, 40 g of sodium carboxymethyl starch (disintegrant) and 10 g of magnesium stearate (lubricant) were added and mixed thoroughly. The final mixture was loaded into a tablet press hopper and compressed into 10,000 tablets, each containing 5 mg of Daoshuilianin A.

### Embodiment 4: Preparation of Daoshuilianin A Capsules

50 g of Daoshuilianin A was mixed with 1800 g of a filler mixture (weight ratio of microcrystalline cellulose : lactose = 2:1) in a mixer at 25 rpm for 10 minutes. Then, 50 mL of a 5% (w/v) hydroxypropyl methylcellulose (HPMC) aqueous solution (binder) was added, and mixing continued for 15 minutes to form a soft mass. The mass was granulated through an 80-mesh sieve and dried to control moisture content between 2% and 5%. The dried granules were sized through a 100-mesh sieve. Subsequently, 50 g of crospovidone (disintegrant) and 10 g of magnesium stearate (lubricant) were added to the mixer, and the mixture was blended at 20 rpm for 20 minutes. The final mixture was filled into capsules using a capsule filling machine to produce 10,000 capsules, each containing 5 mg of Daoshuilianin A.

### Embodiment 5: Preparation of Daoshuilianin A Injection

50 g of Daoshuilianin A was placed in a clean vessel, and 5 L of PEG-400 was added. The mixture was stirred until the compound was completely dissolved. The solution was then diluted with Water for Injection (WFI) to a final volume of 50 L. The resulting solution was filtered through a 0.22 µm microporous membrane filter. Under aseptic conditions, the filtrate was aseptically filled and sealed into 5 mL ampoules, yielding a total of 10,000 units. The sealed ampoules were sterilized using moist heat sterilization at 121°C for 20 minutes. The final product consists of 5 mL per ampoule, containing 5 mg of Daoshuilianin A.

The embodiments described above are merely preferred examples of the present invention and are not intended to limit the scope of the present invention. Accordingly, any simple modifications, equivalent changes, or alterations made to the above embodiments based on the technical essence of the present invention, without departing from the technical solutions of the present invention, shall fall within the scope of the technical solutions of the present invention.

## Claims

1. A drastic water-expelling compound having the chemical structure shown in formula (I):

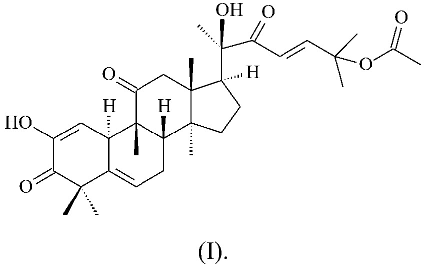

2. A pharmaceutical composition comprising the compound of claim 1.

3. The pharmaceutical composition of claim 2, wherein the pharmaceutical composition further comprises a pharmaceutically acceptable carrier and/or excipient.

4. A method for preparing the compound of claim 1, comprising the following steps:
a. Providing dried rhizomes of *Begonia limprichtii Irmscher*, pulverizing them, extracting with an organic solvent, filtering, and concentrating to obtain a fluid extract;
b. Adding the fluid extract to water, stirring, filtering, washing with water, and drying to obtain a precipitate;
c. Separating the precipitate by silica gel column chromatography, eluting with a 10:0 to 0:10 gradient of petroleum ether-ethyl acetate;
d. Monitoring the fractions by silica gel column chromatography, combining, and concentrating to obtain the compound.

5. The method of claim 4, wherein the organic solvent is any one selected from methanol, ethanol, ethyl acetate, acetone, chloroform, and petroleum ether.

6. The method of claim 4, wherein the ratio of *Begonia limprichtii Irmscher* to the organic solvent is 1 g : 3 to 5 mL.

7. The method of claim 4, wherein in said step (d), the column chromatography fractions are monitored by thin-layer chromatography or high-performance liquid chromatography.

8. Use of the compound of claim 1 or the pharmaceutical composition of claim 2 in the preparation of a medicament for draining ascites.

9. The use of claim 8, wherein the tumor is any one of lung cancer, breast cancer, liver cancer, colon cancer, rectal cancer, and gastric cancer.

10. The use of claim 8, wherein the compound is formulated into a pharmaceutically acceptable dosage form either as a pure compound or as a plant extract containing the compound.
